# EUROPÄISCHE PATENTSCHRIFT

(11) **EP 3 490 511 B1**
(45) Veröffentlichungstag und Bekanntmachung des Hinweises auf die Patenterteilung: **09.09.2020**
(21) Anmeldenummer: 17754093.7
(22) Anmeldetag: 28.07.2017
(51) Int. Cl.: A61F 5/01

(54) **KÖRPERGELENKSTABILISIERUNGSVORRICHTUNG ZUM ADAPTIVEN DÄMPFEN EINER KÖRPERBEWEGUNG**
JOINT STABILIZER FOR ADAPTIVE DAMPING OF A MOVEMENT OF THE BODY
DISPOSITIF DE STABILISATION D'UNE ARTICULATION CORPORELLE POUR L'AMORTISSEMENT ADAPTATIF D'UN MOUVEMENT DU CORPS

(30) Priorität: 29.07.2016 DE 102016114110
(43) Veröffentlichungstag der Anmeldung: 05.06.2019
(73) Patentinhaber: Betterguards Technology GmbH, 10625 Berlin (DE)
(72) Erfinder: BICHLER, Vinzenz, 10777 Berlin (DE); STUMPER, Timo, 12101 Berlin (DE)
(74) Vertreter: Okoampah, Rene
(86) Internationale Anmeldenummer: PCT/EP2017/069207
(87) Internationale Veröffentlichungsnummer: WO 2018/020020

(56) Entgegenhaltungen:
- WO-A1-2013/174989
- WO-A1-2015/177357
- US-B1- 7 402 147

## Beschreibung

### Technisches Gebiet

Die vorliegende Erfindung betrifft eine Körpergelenkstabilisierungsvorrichtung zum adaptiven Dämpfen einer Körperbewegung, umfassend: eine Aufnahme, wobei die Aufnahme mit einem scherverdickenden Medium gefüllt ist, einen relativ zu der Aufnahme beweglichen Zugkörper, wobei der Zugkörper mit einem Körperbereich des Anwenders verbindbar ist, welcher relativ zu einem anderen Körperbereich des Anwenders bewegbar ist, an welchen die Aufnahme befestigbar ist, einen in der Aufnahme verschiebbar angeordneten Wirkkörper, zum Interagieren mit dem scherverdickenden Medium, und ein Verbindungselement zum Übertragen von Kräften zwischen der Aufnahme und dem Zugkörper.

### Stand der Technik

Es ist bekannt Körpergelenke, Muskeln und Sehnen mittels Vorrichtungen, welche eine adaptive Bewegungsbegrenzung ermöglichen zu stabilisieren. Unter anderem wird das adaptive Verhalten solcher Vorrichtungen dadurch erreicht, dass sich zwei Körper relativ zueinander bewegen, wobei sich zwischen den Körpern ein scherverdickendes Fluid befindet. Die sich gegenüberliegenden Flächen der Körper bilden dabei Scherflächen, welche auf Grund der Relativbewegung Scherkräfte in das scherverdickende Fluid einleiten. Umso größer die Scherkräfte sind umso zähflüssiger verhält sich das scherverdickende Fluid. Ab einer definierten Schergeschwindigkeit erfährt das scherverdickende Fluid einen Schersprung, durch welchen der Grad der Viskosität rapide zunimmt.

Die Vorrichtungen werden zwischen zwei Körperstellen eines Anwenders fixiert. Dabei bildet ein Scherkörper der Vorrichtung eine Aufnahme, welche mit dem scherverdickenden Fluid gefüllt ist. Der andere Scherkörper bildet einen Auszugskörper, welcher in der Aufnahme bewegbar angeordnet ist. Werden über die beiden Körperstellen des Anwenders physiologische Kräfte, das heißt für das entsprechend zu stabilisierende Körperteil unkritische Kräfte, in die Vorrichtung eingeleitet, so wird auf Grund des flüssigen Zustands des scherverdickenden Fluids eine Relativbewegung der Aufnahme und des Auszugskörper und damit eine Bewegung des zu stabilisierenden Körperteils zugelassen.

Werden hingegen unphysiologische Kräfte, das heißt für das entsprechend zu stabilisierende Körperteil kritische Kräfte, in die Vorrichtung eingeleitet, rufen die von den Scherflächen der Aufnahme und des Auszugskörpers ausgehenden Scherkräfte eine Scherverfestigung des scherverdickenden Fluids hervor, durch welche eine Relativbewegung zwischen dem Auszugskörper und der Aufnahme nur noch unter sehr hohem Kraftaufwand möglich ist.

Eine solche Vorrichtung ist beispielsweise aus der WO 2013/174989 A1 bekannt, welche eine orthopädische Vorrichtung zur Begrenzung der Bewegung eines zwischen einem ersten und einem zweiten Körperbereich angeordneten Gelenks gemäß des Oberbegriffs von Anspruch 1 zeigt.

Die Gelenke im menschlichen Körper haben sehr unterschiedliche physiologische Bewegungsfreiräume und können sehr unterschiedliche Kräfte umlenken. So sind zum Beispiel beim Beugen des Knies die physiologische Relativbewegung zwischen Ober- und Unterschenkel und die entsprechende Kraft sehr viel größer als zum Beispiel beim Beugen eines Fingers. Nach dem Stand der Technik besteht zwischen den beiden verbundenen Körperteilen eine direkte (lineare) Verbindung. Dies hat zu Folge, dass zum Beispiel bei sehr großen Bewegungen sehr große Vorrichtungen mit Platz für eine entsprechend große Auslenkung gebaut werden müssen, die unter Umständen schlecht anzubringen oder für die Nutzer unbequem sind. Bei sehr kleinen biomechanischen Auslenkungen hingegen ist die Konstruktion einer entsprechend filigranen Vorrichtung technisch schwer zu realisieren, da die zur Verfügung stehende Strecke zur Entfaltung der Schutzwirkung verhältnismäßig gering ist.

### Darstellung der Erfindung

Ausgehend von dem bekannten Stand der Technik ist es eine Aufgabe der vorliegenden Erfindung, eine Körpergelenkstabilisierungsvorrichtung zum adaptiven Dämpfen einer Körperbewegung anzugeben, welche die absolute Größe der relativen Bewegung der Körperteile von der Größe der Körpergelenkstabilisierungsvorrichtung entkoppelt, so dass letztere weitgehend frei gestaltet werden kann und auch gleiche Vorrichtungen an unterschiedlichen Gelenken verwendet werden können.

Diese Aufgabe wird mittels einer Körpergelenkstabilisierungsvorrichtung mit den Merkmalen des Anspruchs 1 gelöst. Vorteilhafte Ausgestaltungen ergeben sich aus den Unteransprüchen.

Entsprechend wird eine Körpergelenkstabilisierungsvorrichtung zum adaptiven Dämpfen einer Körperbewegung angegeben, welche eine Aufnahme, wobei die Aufnahme mit einem scherverdickenden Medium gefüllt ist, einen relativ zu der Aufnahme beweglichen Zugkörper, wobei der Zugkörper mit einem Körperbereich des Anwenders verbindbar ist, welcher relativ zu einem anderen Körperbereich des Anwenders bewegbar ist, an welchen die Aufnahme befestigbar ist, einen in der Aufnahme verschiebbar angeordneten Wirkkörper zum Interagieren mit dem scherverdickenden Medium, und ein Verbindungselement zum Übertragen von Kräften zwischen der Aufnahme und dem Zugkörper, umfasst. Erfindungsgemäß sind die Aufnahme, der Zugkörper und der Wirkkörper über das Verbindungselement miteinander verbunden, wobei das Verbindungselement über mindestens ein Umlenkmittel geführt ist, um die auf das Verbindungselement wirkende Kraft aufzuteilen.

Die Körpergelenkstabilisierungsvorrichtung kann derart am Körper des Anwenders angebracht werden, dass sie mittels der Funktion der adaptiven Bewegungsbegrenzung ein Gelenk eines Anwenders stabilisieren kann.

Je nachdem in welcher Reihenfolge die Aufnahme, der Zugkörper und der Wirkkörper mittels des Verbindungselements miteinander verbunden sind, können unterschiedliche Kraftwandlungsszenarien bereitgestellt werden. Somit ist es möglich den Umfang und die Intensität einer von einem Körperteil ausgehenden Bewegung gegenüber dem Bewegungsumfang und der Bewegungsintensität des Wirkkörpers je nach Anwendungsbereich zu vergrößern oder zu reduzieren. Dadurch können große Körpergelenkstabilisierungsvorrichtungen auch zum Dämpfen von kleinen relativen Körperbewegungen eingesetzt werden. Umgekehrt können kleine Körpergelenkstabilisierungsvorrichtungen dadurch auch bei größeren relativen Körperbewegungen zum Einsatz kommen. Insgesamt lässt sich die absolute Größe der relativen Bewegung der Körperteile von der Größe der Körpergelenkstabilisierungsvorrichtung entkoppeln. Dadurch kann das Einsatzgebiet von Körpergelenkstabilisierungsvorrichtungen deutlich vergrößert werden. Darüber hinaus ergeben sich zusätzliche Freiheiten in Bezug auf die Gestaltung der Körpergelenkstabilisierungsvorrichtung, da diese weniger stark vom Einsatzgebiet der Körpergelenkstabilisierungsvorrichtung beeinflusst wird.

In einer bevorzugten Ausführungsform weist das Verbindungselement ein erstes Ende und ein zweites Ende auf, wobei das mindestens eine Umlenkmittel zwischen dem ersten Ende und dem zweiten Ende an dem Verbindungselement beweglich geführt ist. Dabei bilden das erste Ende, das zweite Ende und das Umlenkmittel jeweils einen Kraftangriffspunkt zum Übertragen einer Kraft zwischen dem Verbindungselement und der Aufnahme, dem Zugkörper und dem Wirkkörper, wobei die Aufnahme, der Zugkörper und der Wirkkörper jeweils mit einem Kraftangriffspunkt fest verbunden sind. Die Zuordnung der Aufnahme, des Zugkörpers und des Wirkkörpers zu dem entsprechenden Kraftangriffspunkt hängt dabei vom Einsatzgebiet der Körpergelenkstabilisierungsvorrichtung ab und variiert je nachdem ob die über den Zugkörper in die Körpergelenkstabilisierungsvorrichtung einleitbare Kraft vergrößert oder reduziert werden soll.

In einer bevorzugten Weiterbildung ist das erste Ende des Verbindungselements mit dem Wirkkörper oder dem Zugkörper und das zweite Ende des Verbindungselements mit der Aufnahme oder dem Zugkörper verbunden.

Wenn beispielsweise das Verbindungselement an dem ersten Ende mit dem Wirkkörper und an dem zweiten Ende mit der Aufnahme verbunden ist, wobei der Zugkörper an dem Verbindungselement zwischen dem ersten Ende und dem zweiten Ende mittels des Umlenkmittels beweglich geführt ist, ist es möglich, dass der Wirkkörper einen größeren Weg durch das scherverdickende Medium zurücklegt, als der Weg, um welchen der Zugkörper infolge einer äußeren Kraft ausgelenkt wird. Insbesondere kann so das Verhältnis des Weges des Wirkkörpers zu dem Weg des Zugkörpers 2:1 betragen. Insgesamt verhält sich die Körpergelenkstabilisierungsvorrichtung deutlich sensibler und reagiert bereits auf kleine Bewegungen des Zugkörpers.

Auf diese Weise kann sichergestellt werden, dass wenn sich der Körperbereich des Anwenders, an welchem die Aufnahme befestigt ist, gegenüber dem Körperbereich des Anwenders, an welchem der Zugkörper befestigt ist, nur geringfügig relativ zueinander bewegen, der Umfang der Bewegung im Wesentlichen verdoppelt werden kann, so dass eine möglichst große Relativbewegung des Wirkkörpers gegenüber der Aufnahme erzeugt wird. Dadurch kann sichergestellt werden, dass auch bei einem kleinen Bewegungseintrag in die Körpergelenkstabilisierungsvorrichtung eine ausreichende Interaktion zwischen dem Wirkkörper und dem scherverdickenden Medium hervorgerufen wird.

Darüber hinaus kann die über den Zugkörper in die Körpergelenkstabilisierungsvorrichtung eingeleitete Kraft auf den Wirkkörper und die Aufnahme zu im Wesentlichen gleichen Teilen aufgeteilt werden. Dies erlaubt eine kleinere Bauweise der Aufnahme und des Wirkkörpers. Insbesondere kann die Aufnahme somit flacher ausgeführt werden. Beispielsweise können dünnere Wandstärken für die Aufnahme gewählt werden.

Wird an dem Zugkörper beispielsweise mit einer Kraft von 1000 N gezogen, so wirkt eine Kraft von 500 N jeweils auf den Wirkkörper und die Aufnahme.

Des Weiteren ist auch die Geschwindigkeit, welche der Wirkkörper erfährt, wenn der Zugkörper ausgelenkt wird, größer als die Geschwindigkeit, mit welcher der Zugkörper ausgelenkt wird. Dabei kann der Betrag der Geschwindigkeit des Wirkkörpers bei einer Auslenkung des Zugkörpers im Wesentlichen doppelt so groß sein, wie der Betrag der Geschwindigkeit, mit welcher der Zugkörper ausgelenkt wird.

Somit ist es möglich, auch bei auf die Körpergelenkstabilisierungsvorrichtung wirkenden, vergleichsweise langsamen Körperbewegungen, ein Erreichen einer kritischen Geschwindigkeit des Wirkkörpers zu erzielen, bei welcher das scherverdickende Medium beginnt, sich im Bereich der Scherflächen des Wirkkörpers zu verfestigen. Das Umlenkmittel lenkt das von dem Wirkkörper kommende Verbindungselement um hin zu der Aufnahme.

Wenn alternativ das Verbindungselement an einem ersten Ende mit dem Zugkörper verbunden ist und an einem zweiten Ende mit der Aufnahme verbunden ist, wobei der Wirkkörper an dem Verbindungselement zwischen dem ersten Ende und dem zweiten Ende mittels des Umlenkmittels beweglich geführt ist, ist es möglich, den Weg, den der Wirkkörper in der Aufnahme zurücklegt, gegenüber dem Weg, welchen der Zugkörpers aufgrund einer einwirkenden äußeren Kraft zurücklegt, zu verkleinern. Insbesondere ist es möglich, dass der Wirkkörper sich infolge einer Auslenkung des Zugkörpers gegenüber diesem in einem Verhältnis von 1:2 bewegt. Das heißt, der Wirkkörper legt innerhalb der Aufnahme nur die Hälfte des Weges zurück, um welchen der Zugkörper aufgrund einer äußeren Kraft bewegt wird.

Eine derartige Körpergelenkstabilisierungsvorrichtung eignet sich für Körperbereiche des Anwenders, welche große Relativbewegungen zulassen. Die Reduzierung des Weges, welchen der Wirkkörper innerhalb der Aufnahme zurücklegt, ermöglicht das Verwenden einer verhältnismäßig klein dimensionierten Aufnahme trotz eines großen Bewegungsbereichs der relativ zueinander bewegbaren Körperbereiche des Anwenders.

Darüber hinaus bewirkt das Umlenkmittel, dass die auf den Wirkkörper wirkende Kraft gegenüber der auf den Zugkörper wirkenden Kraft im Wesentlichen doppelt so groß ist.

Ferner kann die Geschwindigkeit des Wirkkörpers gegenüber der Geschwindigkeit, mit welcher sich der Zugkörper und die Aufnahme relativ zueinander bewegen, reduziert werden. Insbesondere kann die Geschwindigkeit, mit welcher sich der Wirkkörper bewegt, gegenüber der Relativgeschwindigkeit zwischen Zugkörper und Aufnahme halb so groß sein. Dadurch ist es beispielsweise möglich, schnelle physiologische Bewegungen zwischen zwei mittels der Körpergelenkstabilisierungsvorrichtung stabilisierten Körperbereichen zuzulassen. Dadurch ist der Einsatz kleinerer Aufnahmen möglich. Es ist ausreichend, die Aufnahme und den Wirkkörper für Geschwindigkeiten zu dimensionieren, die im Wesentlichen halb so groß sind, wie die Relativgeschwindigkeit zwischen Zugkörper und Aufnahme.

In einer bevorzugten Ausführungsform lenkt das Umlenkmittel (70) das Verbindungselement (50) mindestens einmal um einen Winkel zwischen 150° bis 190° um. In einer weiteren bevorzugten Ausgestaltung lenkt das Umlenkmittel das Verbindungselement mindestens einmal um im Wesentlichen 180° um. Dadurch kommt dem Umlenkmittel die Funktion eines Kraftwandlers zu. So kann die Kraft an den beiden Enden des Verbindungselements jeweils nur die Hälfte der Kraft betragen, welche auf den Körper wirkt, an dem das Umlenkmittel befestigt ist.

Beispielsweise kann das Umlenkmittel in Form eines Umlenkkeils ausgebildet sein. Dabei kann der Umlenkkeil eine umlaufende Furche oder Rille aufweisen, durch welche das Verbindungselement gleiten kann.

In einer bevorzugten Ausführungsform ist der Zugkörper außerhalb der Aufnahme angeordnet. Entsprechend kann der Zugkörper mit einem Körperteil verbunden werden, und die Aufnahme mit einem anderen Körperteil, welches relativ zu dem einen Körperteil bewegbar ist. Darüber hinaus weist die Aufnahme mindestens eine Öffnung auf, durch welche das Verbindungselement vom Inneren der Aufnahme nach außen hin zu dem Zugkörper führt.

In einer Weiterbildung weist das Umlenkmittel ein Material mit einem geringeren Reibungskoeffizienten auf. Dadurch ist es möglich, den Widerstand, welcher auf die Gleitreibung zwischen dem Verbindungselement und dem Umlenkmittel zurückzuführen ist, gering zu halten. Dadurch kann beim Aufstellen eines Kräftegleichgewichts über das erste Ende des Verbindungselements, das zweite Ende des Verbindungselements, und den Teil des Verbindungselements, der mit dem Umlenkmittel in Kontakt steht, die Reibungskraft nahezu vernachlässigt werden. Dies kann weiter begünstigt werden, indem auch das Material des Verbindungselements einen geringen Reibungskoeffizienten aufweist.

In einer weiter bevorzugten Ausführungsform umfasst das Umlenkmittel mindestens eine Umlenkrolle. Bei der Umlenkrolle handelt es sich um einen Kraftwandler, welcher ein Rad oder eine Kreisscheibe umfassen kann, welches auf einer Achse gelagert ist. Mittels der Umlenkrolle kann das Verbindungselement reibungsarm geführt und umgelenkt werden.

In einer weiteren bevorzugten Weiterbildung ist das Verbindungselement zugsteif ausgebildet. Dadurch ist es möglich, dass beim Einleiten einer Zugkraft über das Umlenkmittel in das Verbindungselement das erste Ende des Verbindungselements und das zweite Ende des Verbindungselements jeweils eine Zugkraft übertragen wird. Dadurch eignet sich die Körpergelenkstabilisierungsvorrichtung für Zugbelastungen, bei welchen der Wirkkörper über eine Zugkraft ausgelenkt wird.

In einer weiter bevorzugten Ausgestaltung ist das Verbindungselement in Form eines länglichen, flexiblen Zugelements ausgebildet. Dadurch ist das Verbindungselement in der Lage, der Geometrie des Umlenkmittels zu folgen.

In einer weiter bevorzugten Ausführungsform weist das Verbindungselement Fasern, einen Riemen, einen Gurt, ein Seil, ein Kabel und/oder einen Draht auf. Dadurch ist es möglich, hohe Kräfte zwischen dem Wirkkörper, dem Zugkörper und der Aufnahme zu übertragen und gleichzeitig eine Flexibilität des Verbindungselements bereitzustellen.

In einer Weiterbildung ist das Verbindungselement integral mit dem Wirkkörper ausgebildet ist. Dabei kann der Teil des Verbindungselements, welcher sich in Kontakt mit dem scherverdickenden Medium befindet, den Wirkkörper bilden. Dabei bildet der Teil der Oberfläche des Verbindungselements, welcher mit dem scherverdickenden Medium in Kontakt steht die Scherfläche, welche bei einer Relativbewegung zwischen der Aufnahme und dem Verbindungselement Scherkräfte in das scherverdickende Medium einleitet.

Das längliche Verbindungselement legt mit seinem Durchmesser die Abmessungen der Aufnahme fest. Dadurch ist es möglich Aufnahmen und damit Körpergelenkstabilisierungsvorrichtungen mit besonders kleinen Abmessungen zu bauen.

In einer bevorzugten Ausgestaltung umfasst das Umlenkmittel mindestens zwei Umlenkrollen zum Umlenken des Verbindungselements, wobei die eine Umlenkrolle am Zugkörper und die andere Umlenkrolle am Wirkkörper oder der Aufnahme angeordnet ist. Je nachdem ob das zweite Ende des Verbindungselements am Wirkkörper oder am Zugkörper befestigt ist, kann der Weg, mit welchem der Wirkkörper aufgrund einer Auslenkung des Zugkörpers bewegt wird, um den Faktor 1,5 verkürzt oder verlängert werden.

Wird das Verbindungselement von der Aufnahme über eine Umlenkrolle am Wirkkörper, über eine Umlenkrolle am Zugkörper und schließlich zum Wirkkörper geführt und daran befestigt, kann die Auslenkung des Wirkkörpers, welche auf eine Relativbewegung zwischen dem Zugkörper und der Aufnahme zurückzuführen ist, gegenüber dieser Relativbewegung reduziert werden.

Ist das Verbindungselement am ersten Ende an der Aufnahme befestigt und läuft über eine an dem Zugkörper angeordnete Umlenkrolle, weiter über eine am Wirkkörper angeordnete Umlenkrolle, hin zu dem Zugkörper, wobei das erste Ende des Verbindungselements an diesem befestigt ist, so kann eine Auslenkung des Wirkkörpers, welche auf eine Relativbewegung zwischen dem Zugkörper und der Aufnahme zurückzuführen ist, gegenüber dieser vergrößert werden.

In einer Weiterbildung ist die Interaktion zwischen dem Wirkkörper und dem scherverdickenden Medium derart konfiguriert, dass wenn eine Kraft mit einer Geschwindigkeit unterhalb eines Schwellwerts auf den Wirkkörper wirkt, der Wirkkörper durch das scherverdickende Medium bewegbar ist, und das wenn eine Kraft mit einer Geschwindigkeit größer/gleich eines Schwellwerts auf den Wirkkörper wirkt, das scherverdickende Medium die Bewegung des Wirkkörpers hemmt.

In einer weiter bevorzugten Ausgestaltung umfasst das scherverdickende Medium Festkörper, insbesondere Polymere oder Pulver und/oder ein Fluid, insbesondere eine Paste oder ein Gel, oder eine Kombination daraus.

Unter scherverdickenden Medien sind im Allgemeinen und insbesondere in der vorliegenden Anmeldung Copolymerdispersionen zu verstehen, wie sie beispielsweise in der DE 30 25 562 A1, der DE 34 33 085 A1 und der DE 39 17 456 A1 gezeigt sind. Die Dispersionen setzen sich beispielsweise aus Emulsionscopolymerisaten und Metallsalzen zusammen. Die Emulsionscopolymerisate können beispielsweise aus 1 bis 10 Gew.-% monoolefinisch ungesättigten Mono- und/oder Dicarponsäuren, wie Acryl-, Methacryl-, Malein- und/oder Fumarsäure, 99 bis 90 Gew.-% andere olefinisch ungesättigte Monomere, wie Styrol, C1-C6-Alkylacrylate, wie Methylmethacrylat, und 5 bis 30 Gew.-% eines Carponsäurealylester-Monomers mit zwei oder mehr copolymerisierbaren Doppelbindungen, wie beispielsweise Diallylphthallat polymerisiert werden.

Als Metallsalze werden in der Regel 0,1 bis 30 Gew.-% bezogen auf die Copolymerisate an Metalloxiden, -Hydroxiden, -Halogeniden, - Carbonaten, -Hydrogensulfaten, -Sulfaten und/oder-Phosphaten zugesetzt. Weiterhin enthalten die scherverdickende Flüssigkeiten Verdünnungsmittel wie Alkohole, Glykole, Di- und Triglykole, Formamide und/oder Wasser. Für eine detailliertere Zusammensetzung des scherverdickenden Fluids wird auf DE 30 25 562 A1, DE 39 17 456 A1 sowie die auf EP 1 443 097 A1 verwiesen. Darüber hinaus können Scherverdickende Fluide auch einfache Dispersionen sein, welche ab einem bestimmten Feststoffanteil scherverdickende Eigenschaften aufweisen.

In einer bevorzugten Weiterbildung ist die Aufnahme integral mit einem Rahmen zum Befestigen der Körpergelenkstabilisierungsvorrichtung an einem Körperteil ausgebildet. Der Rahmen kann Schlaufen aufweisen, welche über einen Körperteil, zum Beispiel einen Arm, gezogen werden können. Alternativ kann der Rahmen auch Flansche aufweisen, welche mittels eines Tapes und/oder eines Verbandmaterials am Körper des Anwenders befestigbar sind. Ferner kann der Rahmen auch auf ein Körperteil aufgeklebt werden. Des Weiteren kann der Rahmen auch Laschen aufweisen, die um ein Körperteil des Anwenders gelegt werden können und mittels Klettverschluss fixierbar sind.

Die Aufnahme ist in Form einer Kammer in dem Rahmen integriert. Darüber hinaus kann auch der Zugkörper einen Rahmen aufweisen, um den Zugkörper an dem Körper des Anwenders zu befestigen.

### Kurze Beschreibung der Figuren

Bevorzugte weitere Ausführungsformen und Aspekte der vorliegenden Erfindung werden durch die nachfolgende Beschreibung der Figuren näher erläutert. Dabei zeigen:
- Figuren 1A, 1B und 1C: schematisch unterschiedliche Ansichten einer Körpergelenkstabilisierungsvorrichtung zum adaptiven Dämpfen einer Körperbewegung, mittels welcher eine Vergrößerung der Bewegung des Wirkkörpers möglich ist,
- Figuren 2A, 2B und 2C: schematisch unterschiedliche Ansichten einer Körpergelenkstabilisierungsvorrichtung zum adaptiven Dämpfen einer Körperbewegung, mittels welcher eine Reduktion der Bewegung des Wirkkörpers möglich ist,
- Figuren 3A, 3B und 3C: schematisch unterschiedliche Ansichten einer Körpergelenkstabilisierungsvorrichtung zum adaptiven Dämpfen einer Körperbewegung, mittels welcher eine Vergrößerung der Bewegung des Wirkkörpers möglich ist,
- Figuren 4A, 4B und 4C: schematisch unterschiedliche Ansichten einer Körpergelenkstabilisierungsvorrichtung zum adaptiven Dämpfen einer Körperbewegung, mittels welcher eine Reduktion der Bewegung des Wirkkörpers möglich ist,
- Figuren 5A, 5B und 5C: schematisch unterschiedliche Ansichten einer Körpergelenkstabilisierungsvorrichtung zum adaptiven Dämpfen einer Körperbewegung, mittels welcher eine Reduktion der Bewegung des Wirkkörpers möglich ist,
- Figuren 6A und 6B: schematisch unterschiedliche perspektivische Ansichten einer Körpergelenkstabilisierungsvorrichtung zum adaptiven Dämpfen der Bewegung eines Handgelenks,
- Figur 6C: eine Detailansicht der Körpergelenkstabilisierungsvorrichtung aus Figur 6A,
- Figur 6D: eine Schnittansicht der Körpergelenkstabilisierungsvorrichtung entlang der Schnittlinie H-H aus Figur 6A,
- Figuren 7A, 7B und 7C: schematisch unterschiedliche Ansichten eines Umlenkkeils, und
- Figuren 8A, 8B und 8C: schematisch unterschiedliche Ansichten einer Umlenkrolle.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente mit identischen Bezugszeichen bezeichnet. Um Redundanzen zu vermeiden, wird auf eine wiederholte Beschreibung dieser Elemente in der nachfolgenden Beschreibung teilweise verzichtet.

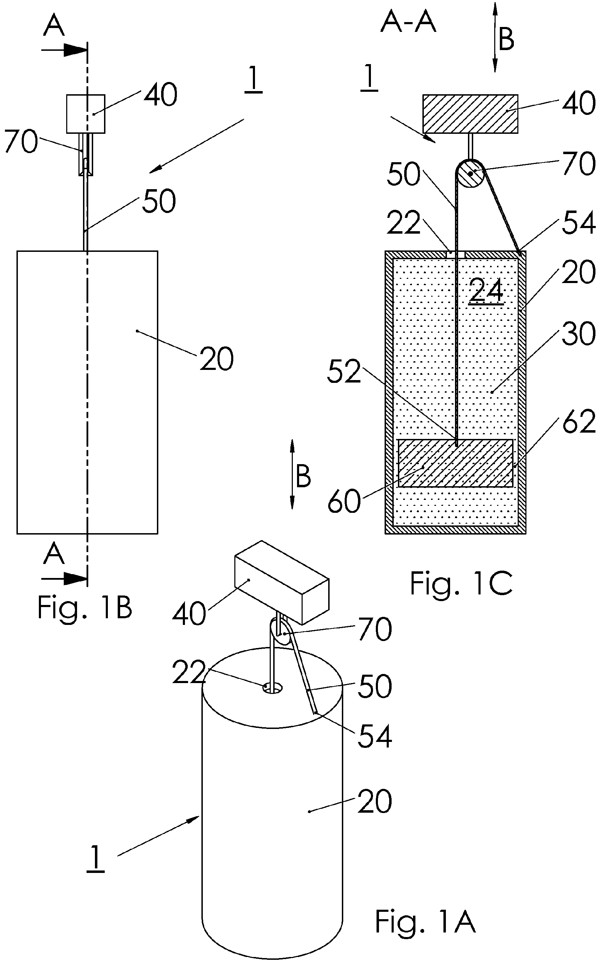

Figuren 1A, 1B und 1C ist eine Körpergelenkstabilisierungsvorrichtung 1 zum adaptiven Dämpfen einer Körperbewegung zu entnehmen. Die Körpergelenkstabilisierungsvorrichtung 1 weist eine zylindrische Aufnahme 20 zum Aufnehmen eines scherverdickenden Mediums und eines Wirkkörpers auf. Auf einer Seite der Aufnahme 20 ist eine Öffnung 22 in Bewegungsrichtung B angeordnet. Ein Verbindungselement 50 zum Verbinden des Wirkkörpers, des Zugkörpers 40 und der Aufnahme 20 verläuft durch die Öffnung 22. Alternativ kann die Aufnahme auch einen im Wesentlichen rechteckigen Querschnitt aufweisen, wobei die äußeren Wandungen der Körpergelenkstabilisierungsvorrichtung 1 Krümmungen zur Anpassung an die Körpergeometrie des Anwenders aufweisen können.

Außerhalb der Aufnahme 20 ist der Zugkörper 40 angeordnet. Dabei kann der Zugkörper 40 an einem Körperbereich eines Anwenders angeordnet werden, wobei die Aufnahme 20 an einem anderen Körperbereich des Anwenders angeordnet werden kann. Die Körpergelenkstabilisierungsvorrichtung 1 ist dadurch in der Lage, Bewegungen zwischen diesen beiden Körperbereichen adaptiv zu dämpfen.

An dem Zugkörper 40 ist ein Umlenkmittel 70 in Form einer Umlenkrolle angeordnet. Das Verbindungselement 50 verläuft über das Umlenkmittel. Das Umlenkmittel 70 lenkt das Verbindungselement 50 derart um, dass das Verbindungselement 50 zwei Abschnitte aufweist, welche beide im Wesentlichen in Richtung der Hauptbewegungsrichtung B verlaufen.

Ein zweites Ende 54 des Verbindungselements 50 ist an der äußeren Oberfläche der Aufnahme 20, auf der gleichen Seite, auf der auch die Öffnung 22 angeordnet ist, befestigt. Das Verbindungselement 50 ist faserförmig, das heißt in Form einer Schnur ausgebildet. Alternativ kann das Verbindungselement auch in Form eines Gurts, Riemens, Kabels und/oder Drahts ausgebildet sein.

Die Aufnahme 20, der Zugkörper 40 und das Umlenkmittel 70 sind aus Polypropylen (PP) gefertigt. Alternativ können die Aufnahme und der Zugkörper auch aus einem andren Kunststoff oder einem Metall wie zum Beispiel Aluminium oder Keramik gefertigt sein.

Figur 1C ist eine Schnittansicht der Körpergelenkstabilisierungsvorrichtung 1 entlang der Schnittlinie A-A aus Figur 1B. Insbesondere ist Figur 1C der Innenraum 24 der Aufnahme 20 zu entnehmen. Der Innenraum 24 ist mit einem scherverdickenden Medium 30 gefüllt. Das in Figur 1C gezeigte scherverdickende Medium ist ein dilatantes Fluid. Alternativ kann auch ein scherverdickender Feststoff verwendet werden. Des Weiteren kann auch Sand als Medium zum Einsatz kommen.

Darüber hinaus ist im Innenraum 24 der Aufnahme 20 ein Wirkkörper 60 angeordnet. Der Wirkkörper 60 ist an dem ersten Ende 52 des Verbindungselements 50 befestigt und kann durch eine Relativbewegung zwischen der Aufnahme 20 und dem Zugkörper 40, insbesondere einer Bewegung, bei welcher sich die Aufnahme 20 und der Zugkörper 40 auseinander bewegen, in dem Innenraum 24 in Bewegungsrichtung B verschoben werden. Wird der Wirkkörper 60 von dem Verbindungselement 50 in Richtung der Öffnung 22 gezogen, so wird der Wirkkörper 60 von dem scherverdickenden Medium 30 umströmt. Der Wirkkörper 60 weist eine Scherfläche 62 auf, welche die Oberfläche des Wirkkörpers 60 bildet, gegenüber welcher das scherverdickende Medium strömt.

Bewegt sich der Wirkkörper 60 unterhalb einer kritischen Geschwindigkeit durch das scherverdickende Medium 30, so kann der Wirkkörper 60 das scherverdickende Medium 30 verdrängen und sich ungehindert hin zu der Öffnung 22 bewegen. Werden der Zugkörper 40 und die Aufnahme 20 derart schnell, das heißt ruckartig, auseinander bewegt, dass der Wirkkörper 60 mit einer Geschwindigkeit größer/gleich einer kritischen Geschwindigkeit durch das scherverdickende Medium 30 bewegt, so beginnt sich das scherverdickende Medium im Bereich der Scherflächen 62 des Wirkkörpers 60 zu verfestigen. Dadurch wird die Beweglichkeit des Wirkkörpers 60 in Richtung der Aufnahme 22 in Abhängigkeit der Geschwindigkeit, mit welcher sich der Wirkkörper 60 bewegt, eingeschränkt bzw. verhindert.

Die Konfiguration des Verbindungselements 50 und des Umlenkmittels 70 ermöglicht eine Übersetzung des Bewegungsweges, von dem Zugkörper 40 auf den Wirkkörper 60. Die in den Figuren 1A, 1B und 1C gezeigte Körpergelenkstabilisierungsvorrichtung 1 kann ein Übersetzungsverhältnis des Weges und der Geschwindigkeit von 1:2 zwischen dem Zugkörper 40 und dem Wirkkörper 60 ermöglichen. Das heißt, wird der Zugkörper 40 aufgrund einer Auseinanderbewegung der beiden Körperbereiche des Anwenders um einen Weg X voneinander beabstandet, so legt der Wirkkörper 60 relativ zur Aufnahme 20 den doppelten Weg, das heißt, 2X, zurück. Das Gleiche gilt für die Geschwindigkeit, so dass wenn der Zugkörper 40 mit der Geschwindigkeit V von der Aufnahme 20 weg bewegt wird, der Wirkkörper 60 sich mit der doppelten Geschwindigkeit, das heißt, 2V, gegenüber der Aufnahme 20 bewegt.

Was die Kräftebilanz betrifft, so verhalten sich Zugkörper 40 und Wirkkörper 60 im Verhältnis 2:1. Das heißt, wirkt bei der Beabstandung der Körperbereiche des Anwenders eine Kraft F auf den Zugkörper 40, so wirkt lediglich die Hälfte der Kraft F, das heißt 1/2 F, auf den Wirkkörper 60.

Das Umlenkmittel 70 dient entsprechend als Kraftwandler, durch welchen das Einsatzgebiet der Körpergelenkstabilisierungsvorrichtung 1 sowie der Dimensionierung der Komponenten der Körpergelenkstabilisierungsvorrichtung 1 beeinflusst werden können. Die Vergrößerung des Weges bzw. der Geschwindigkeit, mit welcher sich der Wirkkörper 60 gegenüber der Aufnahme 20 bewegt, kann beispielsweise für Anwendungsbereiche hilfreich sein, in welchen die zu stabilisierenden Körperbereiche des Anwenders nur vergleichsweise kleine und/oder langsame Bewegungen aufweisen, für welche es schwerfällt, den Effekt der Scherverfestigung in der Aufnahme 20 optimal auszunutzen.

Der Wirkkörper ist aus Polypropylen (PP) gefertigt. Alternativ kann der Wirkkörper auch aus einem andren Kunststoff oder einem Metall wie zum Beispiel Aluminium gefertigt sein.

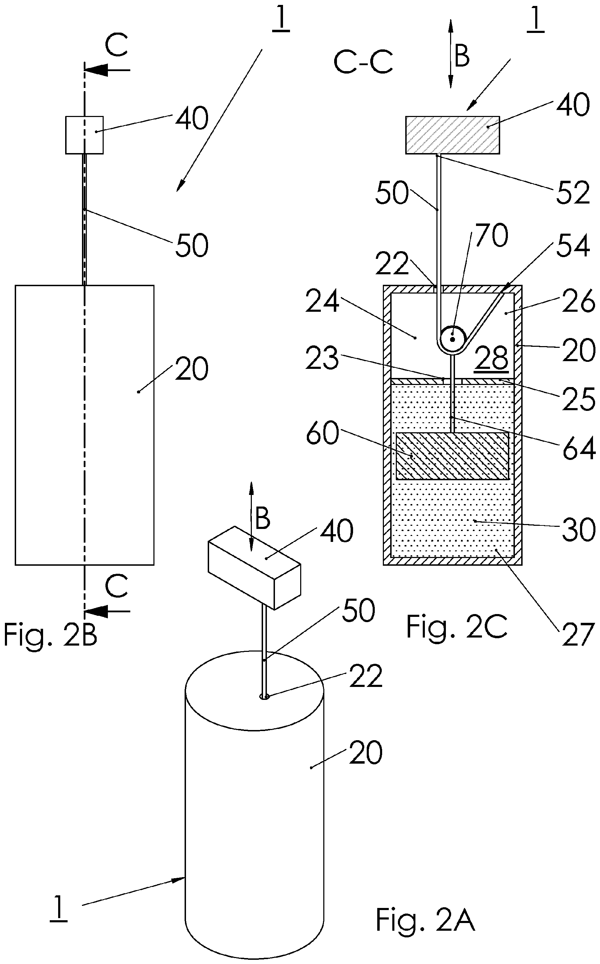

Figuren 2A, 2B und 2C zeigen eine perspektivische Ansicht einer Körpergelenkstabilisierungsvorrichtung 1 zum adaptiven Dämpfen einer Körperbewegung. Die Körpergelenkstabilisierungsvorrichtung 1 aus den Figuren 2A, 2B und 2C unterscheidet sich von der Körpergelenkstabilisierungsvorrichtung aus Figuren 1A, 1B und 1C dadurch, dass außerhalb der Aufnahme 20 nur ein Abschnitt des Verbindungselements 50 und der Zugkörper 40 angeordnet sind.

Figur 2C zeigt eine Schnittansicht der Körpergelenkstabilisierungsvorrichtung 1 entlang der Schnittlinie C-C aus Figur 2B, welcher zu entnehmen ist, dass der Innenraum 24 der Aufnahme 20 mittels einer Trennwand 25 in eine erste Kammer 27 und eine zweite Kammer 28 geteilt ist. Der Wirkkörper 60 ist in der ersten Kammer 27 angeordnet. In der zweiten Kammer 28 ist ein Umlenkmittel 70 in Form einer Umlenkrolle angeordnet, welche über ein Stabelement 64 mit dem Wirkkörper 60 verbunden ist. Dabei ist die Umlenkrolle an dem Stabelement 64 rotierbar gelagert. Das Stabelement verläuft von der ersten Kammer 27 durch eine Öffnung 23 in der Trennwand 25 in die zweite Kammer 28. Bewegungen, welche ausgehend von dem Verbindungselement 50 auf die Umlenkrolle wirken, werden mittels des Stabelements 64 auf den Wirkkörper übertragen. Aufgrund der vorstehend beschriebenen Anordnung wird verhindert, dass das Umlenkmittel mit dem scherverdickenden Medium in Kontakt tritt.

Darüber hinaus sind im Innenraum 24 der Aufnahme zwei Abschnitte des Verbindungselements 50 angeordnet, wobei ein Abschnitt von dem Umlenkmittel 70 hin zur Öffnung 22 verläuft und der andere Abschnitt von dem Umlenkmittel 70 hin zur Innenwand 26 der Aufnahme 20 in der Umgebung der Öffnung 22 verläuft. Die beiden Abschnitte des Verbindungselements 50 verlaufen dabei im Wesentlichen in Bewegungsrichtung B.

Die in den Figuren 2A, 2B und 2C gezeigte Körpergelenkstabilisierungsvorrichtung 1 verhält sich umgekehrt gegenüber der in den Figuren 1A, 1B und 1C gezeigten Körpergelenkstabilisierungsvorrichtung. Das Übersetzungsverhältnis von dem in den Figuren 2A, 2B und 2C gezeigten Zugkörper 40 gegenüber dem Wirkkörper 60 beträgt 2:1 bezogen auf den Weg und die Geschwindigkeit. Das bedeutet, dass bei einem Auseinanderbewegen des Zugkörpers 40 und der Aufnahme 20 der Zugkörper 40 gegenüber dem Wirkkörper 60 den doppelten Weg zurücklegt.

Gleiches gilt für das Verhältnis der Geschwindigkeiten, mit welcher sich der Zugkörper 40 und der Wirkkörper 60 bei einer Auslenkung der Körpergelenkstabilisierungsvorrichtung 1 bewegen. Das heißt, wenn sich der Zugkörper 40 und die Aufnahme 20 auseinanderbewegen, bewegt sich der Zugkörper 40 gegenüber dem Wirkkörper 60 mit einer doppelten Geschwindigkeit. Das Kräfteverhältnis verhält sich umgekehrt. So wirkt bei einer Auslenkung der Körpergelenkstabilisierungsvorrichtung 1, das heißt bei einem Auseinanderbewegen des Zugkörpers 40 und der Aufnahme 20, auf den Aufzugkörper 40 eine Kraft, die halb so groß ist wie die Kraft, die auf den Wirkkörper 60 wirkt.

Bewegen sich beispielsweise die Aufnahme 20 und der Zugkörper 40 auseinander, so dass eine Kraft von 500 N auf den Zugkörper 40 wirkt, wirkt eine Kraft von 1000 N auf den Wirkkörper 60.

Eine derartige Konfiguration des Umlenkmittels 70 und des Verbindungselements 50 kann für Anwendungsbereiche hilfreich sein, in welchen es zwischen den zu stabilisierenden Körperbereichen zu verhältnismäßig großen und/oder schnellen Bewegungen kommt. So lässt die Reduktion des Bewegungsumfangs des Wirkkörpers 60 eine kleinere Dimensionierung der Körpergelenkstabilisierungsvorrichtung 1 zu.

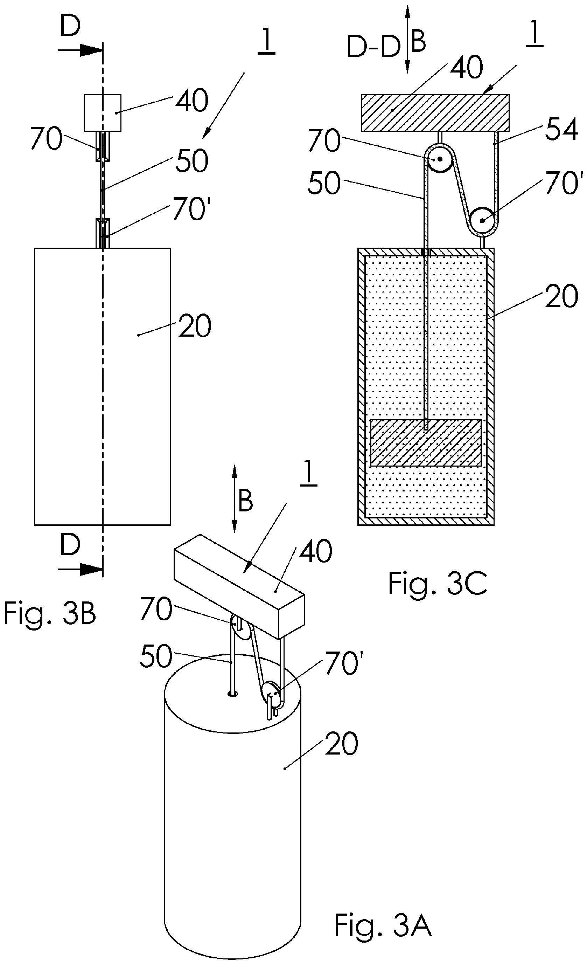

Figuren 3A, 3B und 3C zeigen eine Körpergelenkstabilisierungsvorrichtung 1 zum adaptiven Dämpfen einer Körperbewegung. Die in den Figuren 3A, 3B und 3C gezeigte Körpergelenkstabilisierungsvorrichtung 1 unterscheidet sich dadurch von der in Figuren 1A, 1B und 1C gezeigten Körpergelenkstabilisierungsvorrichtung, dass an der Aufnahme 20 ein weiteres Umlenkmittel 70' angeordnet ist, welches das Verbindungselement zusätzlich umlenkt. Entsprechend weist das Verbindungselement 50 einen dritten Abschnitt auf, welcher nahezu in Bewegungsrichtung B von der an der Aufnahme 20 angeordneten Umlenkrolle 70' hinzu dem Zugkörper 40 verläuft. Ein zweites Ende 54 des Verbindungselements 50 ist am Zugkörper 40 befestigt.

Die in den Figuren 3A, 3B und 3C gezeigte Körpergelenkstabilisierungsvorrichtung 1 weist, auf die Zugkraft bezogen, ein Übersetzungsverhältnis zwischen dem Zugkörper 40 und dem Wirkkörper 60 von 3 zu 1 auf. Das Übersetzungsverhältnis zwischen dem Wirkkörper 60 und der Aufnahme 20 in Bezug auf die Zugkraft beträgt 1 zu 2.

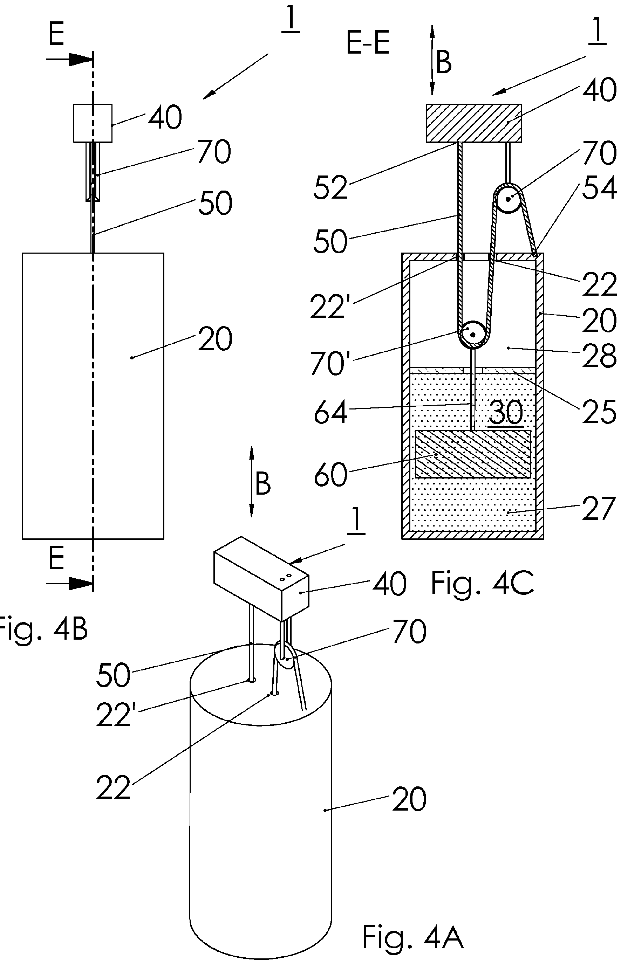

Figuren 4A, 4B, und 4C zeigen eine Körpergelenkstabilisierungsvorrichtung 1 zum adaptiven Dämpfen einer Körperbewegung. Die in den Figuren 4A, 4B, und 4C gezeigte Körpergelenkstabilisierungsvorrichtung 1 unterscheidet sich dadurch von der in den Figuren 1A, 1B und 1C gezeigten Körpergelenkstabilisierungsvorrichtung, dass an dem Wirkkörper 60 ein weiteres Umlenkmittel 70' angeordnet ist, welches das Verbindungselement 50 zusätzlich umlenkt. Entsprechend weist das Verbindungselement 50 einen dritten Abschnitt auf, welcher nahezu in Bewegungsrichtung B verläuft und aus einer weiteren Öffnung 22' aus der Aufnahme 20 heraustritt. Das erste Ende 52 des Verbindungselements 50 ist am Zugkörper 40 befestigt.

Ferner ist Figur 4C zu entnehmen, dass die Aufnahme 20, wie in Figur 2C gezeigt, mittels einer Trennwand 25 in eine erste Kammer 27 und eine zweite Kammer 28 unterteilt ist, um zu verhindern, dass das Umlenkmittel 70' mit dem scherverdickenden Medium 30 in Kontakt tritt. Dabei ist das Umlenkmittel 70' über ein Stabelement 64 mit dem Wirkkörper 60 verbunden.

Die in den Figuren 4A, 4B, und 4C gezeigte Körpergelenkstabilisierungsvorrichtung 1 weist, auf die Zugkraft bezogen, ein Übersetzungsverhältnis zwischen dem Zugkörper 40 und dem Wirkkörper 60 von 3 zu 2 auf. Das Übersetzungsverhältnis zwischen dem Wirkkörper 60 und der Aufnahme 20 in Bezug auf die Zugkraft beträgt 2 zu 1.

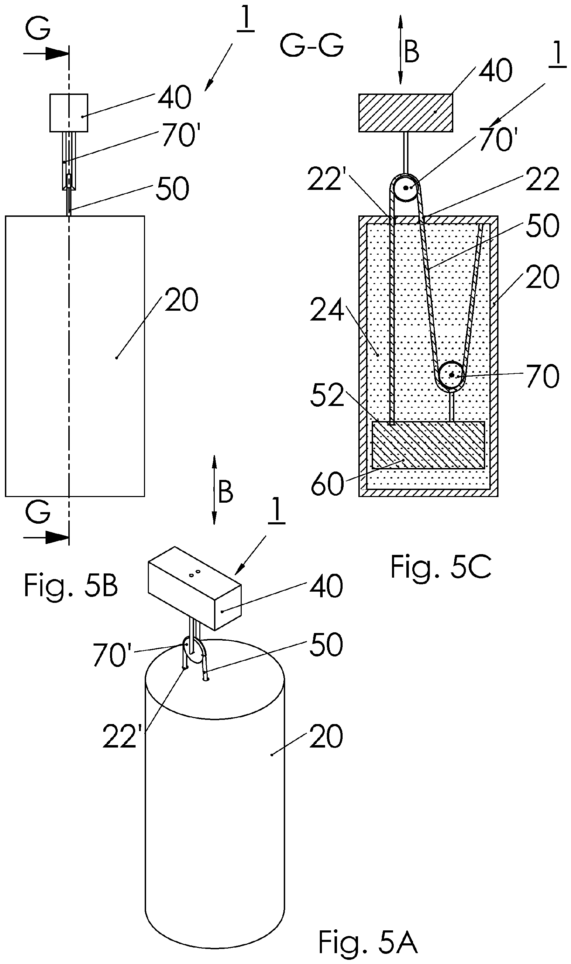

Figuren 5A, 5B, und 5C zeigen eine Körpergelenkstabilisierungsvorrichtung 1 zum adaptiven Dämpfen einer Körperbewegung. Die in den Figuren 5A, 5B, und 5C gezeigte Körpergelenkstabilisierungsvorrichtung 1 unterscheidet sich dadurch von der in den Figuren 2A, 2B und 2C gezeigten Körpergelenkstabilisierungsvorrichtung, dass an dem Zugkörper 40 ein weiteres Umlenkmittel 70' angeordnet ist, welches das Verbindungselement 50 zusätzlich umlenkt. Entsprechend weist das Verbindungselement 50 einen dritten Abschnitt auf, welcher nahezu in Bewegungsrichtung B verläuft und durch eine weitere Öffnung 22' in die Aufnahme 20 eintritt. Das erste Ende 52 des Verbindungselements 50 ist am Wirkkörper 60 befestigt. Darüber hinaus ist der Innenraum 24 der Aufnahme 20 nicht durch eine Trennwand geteilt.

Die in den Figuren 5A, 5B, und 5C gezeigte Körpergelenkstabilisierungsvorrichtung 1 weist, auf die Zugkraft bezogen, ein Übersetzungsverhältnis zwischen dem Zugkörper 40 und dem Wirkkörper 60 von 2 zu 3 auf. Das Übersetzungsverhältnis zwischen dem Wirkkörper 60 und der Aufnahme 20 in Bezug auf die Zugkraft beträgt 3 zu 1.

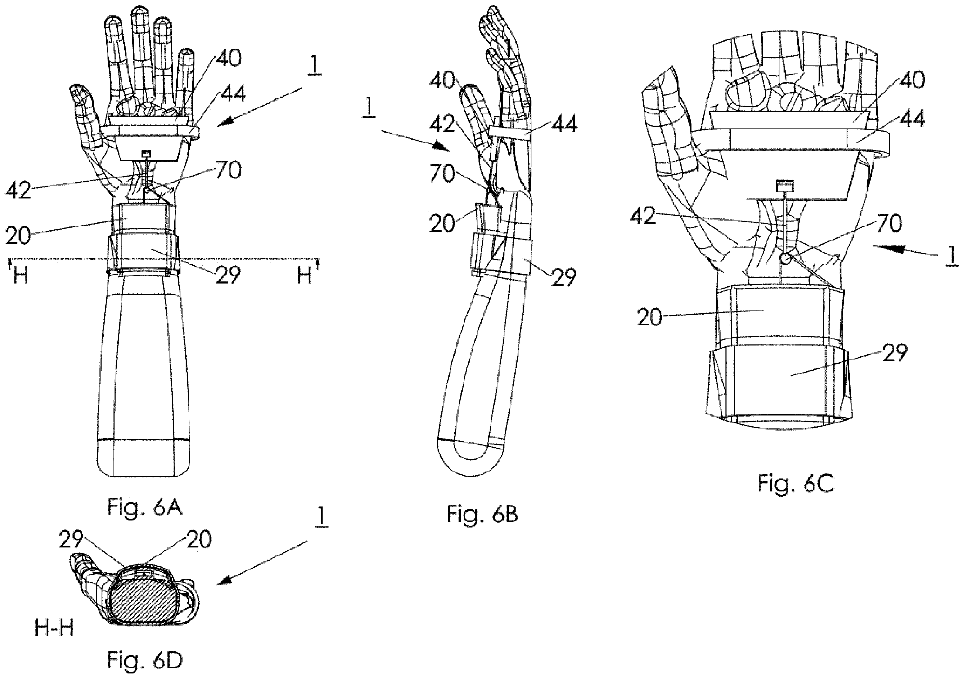

Figuren 6A, 6B, 6C und 6D zeigen eine Körpergelenkstabilisierungsvorrichtung 1 zum adaptiven Dämpfen einer Körperbewegung, welche an einer Hand und einem Unterarm zur Stabilisierung des Handgelenks angebracht ist. Die in den Figuren 6A, 6B und 6C gezeigte Körpergelenkstabilisierungsvorrichtung 1 zeigt Befestigungsschlaufen 29, 44. Die Befestigungsschlaufe 29 dient dazu, die Aufnahme 20 am Unterarm zu befestigen. Die Befestigungsschlaufe 44 dient dazu den Zugkörper 40 an der Hand zu befestigen.

Die Befestigungsschlaufen 29, 44 sind fest mit der Aufnahme 20 beziehungsweise dem Zugkörper 40 verbunden und haften beispielsweise durch eine Klebverbindung an der Aufnahme 20 beziehungsweise dem Zugkörper 40 an. Alternative können die Befestigungsschlaufen auch einstückig mit der Aufnahme beziehungsweise dem Zugkörper gebildet sein. In einer weiteren Alternative halten die Befestigungsschlaufen die Aufnahme und den Zugkörper über dem Prinzip der Haftreibung.

Dabei können die Befestigungsschlaufen mittels eines Klettverschlusses geschlossen werden. Alternativ können die Befestigungsschlaufen auch aus geschlossenen Gummibändern bestehen, wobei die Schlaufen durch ihre elastischen Eigenschaften dafür sorgen, dass die Aufnahme und der Zugkörper am Körper des Anwenders gehalten werden.

In einer weiteren Alternative kann die Befestigungsschlaufe zur Befestigung des Zugkörpers auch durch einen Handschuh ersetzt werden, wobei der Zugkörper in den Handschuh integriert und mit diesem fest verbunden ist.

In einer noch weiteren Alternative können die Befestigungsschlaufen durch einen Verband oder ein Tape ersetzt werden, welche um die Hand beziehungsweise den Unterarm gewickelt werden, um die den Zugkörper beziehungsweise die Aufnahme zu befestigen.

Die in den Figuren 6A, 6B und 6C gezeigte Körpergelenkstabilisierungsvorrichtung 1 zeigt ferner dass der Zugkörper 40 ein Stabelement 42 umfasst, an welchem ein Umlenkmittel 70 befestigt ist. Darüber hinaus ist den Figuren 6A, 6B, 6C und 6D zu entnehmen, dass die Aufnahme 20 an die Form des Unterarms angepasst ist.

Das Funktionsprinzip der in den 6A, 6B, 6C und 6D gezeigten Körpergelenkstabilisierungsvorrichtung 1 entspricht dem der in den Figuren 1A, 1B und 1C gezeigten Körpergelenkstabilisierungsvorrichtung. Entsprechend ist die Körpergelenkstabilisierungsvorrichtung 1 in der Lage, Bewegungen zwischen der Hand und dem Unterarm, das heißt Bewegungen des Handgelenks adaptiv zu dämpfen.

Figur 6D ist eine Schnittansicht der Körpergelenkstabilisierungsvorrichtung 1 entlang der Schnittlinie H-H aus Figur 6A. Figur 6D zeigt die Befestigungsschlaufe 29, welche den Unterarm vollständig umläuft und so die Aufnahme 20 am Unterarm befestigt.

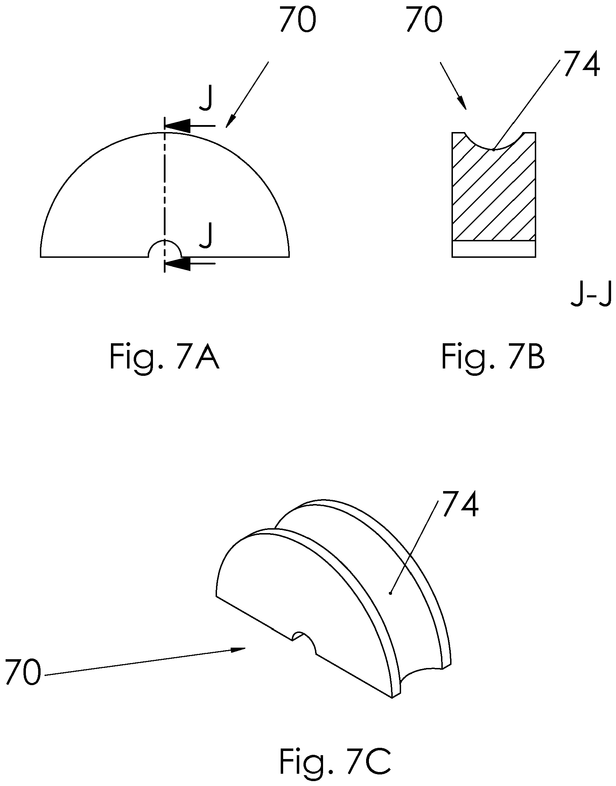

Figur 7C zeigt eine perspektivische Detailansicht eines Umlenkmittels 70 in Form eines Reibkeils. Der Reibkeil ist in der Lage, ein Verbindungselement in etwa um 180° umzulenken. Um das Verbindungselement aufzunehmen und zu führen, weist der Reibkeil eine Laufrille 74 auf. Figur 7A zeigt eine Seitenansicht des Reibkeils. Figur 7B zeigt eine Schnittansicht des Reibkeils entlang der Schnittlinie J-J aus Figur 7A.

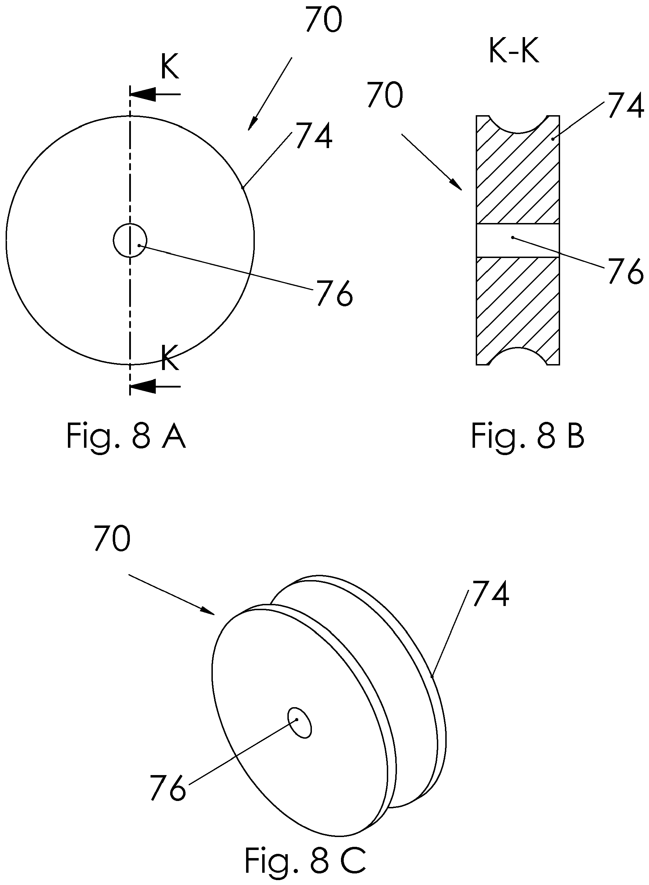

Figur 8C zeigt ein Umlenkmittel 70 in Form einer Umlenkrolle. Die Umlenkrolle weist eine Laufrille 74 zum Aufnehmen und Führen eines Verbindungselements auf. Darüber hinaus weist die Umlenkrolle ein Lager 76 zum rotierbaren Lagern der Umlenkrolle auf. Das Lager 76 kann beispielsweise in Form eines Kugellagers ausgeführt sein. Figur 8A zeigt eine Seitenansicht der Umlenkrolle. Figur 8B zeigt eine Schnittansicht der Umlenkrolle entlang der Schnittlinie K-K aus Figur 7A.

Die in den Figuren 7A bis 8C gezeigten Umlenkmittel sind aus Polypropylen (PP) gefertigt. Alternativ kann der Reibkeil oder die Umlenkrolle auch aus einem anderen Kunststoff gefertigt sein. Darüber hinaus können der Reibkeil oder die Umlenkrolle auch aus Metallen wie zum Beispiel Aluminium, Magnesium, Stahl oder anderen reibungsarmen Materialien gefertigt sein.

Um die in den vorstehenden Figuren dargestellten Körpergelenkstabilisierungsvorrichtungen wieder in eine Ausgangsposition zu bringen, können Rückstellmittel vorgesehen sein. Diese Rückstellmittel können beispielsweise elastisch ausgeführt sein und den Wirkkörper mit der Seite der Aufnahme verbinden, welche der Öffnung gegenüberliegt. Wird der Wirkkörper durch eine einwirkende Kraft aus der Ausgangsposition ausgelenkt, wird das elastische Rückstellmittel gedehnt. Lässt die äußere Kraft sowie die Haltekraft der Scherverfestigung nach, kann das elastische Rückstellmittel aufgrund der zuvor erfahrenen Dehnung den Wirkkörper, das Verbindungselement und den Zugkörper wieder in die Ausgangsposition befördern.

Soweit anwendbar, können alle einzelnen Merkmale, die in den einzelnen Ausführungsbeispielen dargestellt sind, miteinander kombiniert und/oder ausgetauscht werden, ohne den Bereich der Erfindung zu verlassen.

### Bezugszeichenliste

- 1: Körpergelenkstabilisierungsvorrichtung

- 20: Aufnahme
- 22, 22': Öffnung
- 23: Öffnung
- 24: Innenraum
- 25: Trennwand
- 26: Innenwand
- 27: Erste Kammer
- 28: Zweite Kammer
- 29: Befestigungsschlaufe

- 30: Scherverdickendes Medium

- 40: Zugkörper
- 42: Stabelement
- 44: Befestigungsschlaufe

- 50: Verbindungselement
- 52: Erstes Ende
- 54: Zweites Ende

- 60: Wirkkörper
- 62: Scherfläche
- 64: Stabelement

- 70, 70': Umlenkmittel
- 74: Laufrille
- 76: Lager

- B: Bewegungsrichtung

## Patentansprüche

1. Körpergelenkstabilisierungsvorrichtung (1) zum adaptiven Dämpfen einer Körperbewegung, umfassend:
eine Aufnahme (20), wobei die Aufnahme (20) mit einem scherverdickenden Medium (30) gefüllt ist,
einen relativ zu der Aufnahme (20) beweglichen Zugkörper (40), wobei der Zugkörper (40) mit einem Körperbereich des Anwenders verbindbar ist, welcher relativ zu einem anderen Körperbereich des Anwenders bewegbar ist, an welchem die Aufnahme (20) befestigbar ist, einen in der Aufnahme (20) verschiebbar angeordneten Wirkkörper (60), zum Interagieren mit dem scherverdickenden Medium (30), und
ein Verbindungselement (50) zum Übertragen von Kräften zwischen der Aufnahme (20) und dem Zugkörper (40),
**dadurch gekennzeichnet, dass**
die Aufnahme (20), der Zugkörper (40) und der Wirkkörper (60) über das Verbindungselement (50) miteinander verbunden sind, wobei das Verbindungselement (50) über mindestens ein Umlenkmittel (70) geführt ist, um die auf das Verbindungselement (50) wirkende Kraft aufzuteilen.

2. Körpergelenkstabilisierungsvorrichtung (1) gemäß Anspruch 1, **dadurch gekennzeichnet, dass** das Verbindungselement (50) ein erstes Ende (52) und ein zweites Ende (54) aufweist, wobei das mindestens eine Umlenkmittel (70) zwischen dem ersten Ende (52) und dem zweiten Ende (54) an dem Verbindungselement (50) beweglich geführt ist.

3. Körpergelenkstabilisierungsvorrichtung (1) gemäß Anspruch 1 oder 2, **dadurch gekennzeichnet, dass** das erste Ende (52) des Verbindungselements (50) mit dem Wirkkörper (60) oder dem Zugkörper (40) und das zweite Ende (54) des Verbindungselements (50) mit der Aufnahme (20) oder dem Zugkörper (40) verbunden ist.

4. Körpergelenkstabilisierungsvorrichtung (1) gemäß einem der vorstehenden Ansprüche, **dadurch gekennzeichnet, dass** das Umlenkmittel (70) das Verbindungselement (50) mindestens einmal um einen Winkel zwischen 150° bis 190° umlenkt.

5. Körpergelenkstabilisierungsvorrichtung (1) gemäß einem der vorstehenden Ansprüche, **dadurch gekennzeichnet, dass** das Umlenkmittel (70) das Verbindungselement (50) mindestens einmal um im Wesentlichen 180° umlenkt.

6. Körpergelenkstabilisierungsvorrichtung (1) gemäß einem der vorstehenden Ansprüche, **dadurch gekennzeichnet, dass** der Zugkörper (40) außerhalb der Aufnahme (20) angeordnet ist.

7. Körpergelenkstabilisierungsvorrichtung (1) gemäß einem der vorstehenden Ansprüche, **dadurch gekennzeichnet, dass** das Umlenkmittel (70) ein Material mit einem geringen Reibungskoeffizienten aufweist.

8. Körpergelenkstabilisierungsvorrichtung (1) gemäß einem der vorstehenden Ansprüche, **dadurch gekennzeichnet, dass** das Umlenkmittel (70) mindestens eine Umlenkrolle umfasst.

9. Körpergelenkstabilisierungsvorrichtung (1) gemäß einem der vorstehenden Ansprüche, **dadurch gekennzeichnet, dass** das Verbindungselement (50) zugsteif ausgebildet ist.

10. Körpergelenkstabilisierungsvorrichtung (1) gemäß einem der vorstehenden Ansprüche, **dadurch gekennzeichnet, dass** das Verbindungselement (50) in Form eines länglichen, flexiblen Zugelements ausgebildet ist.

11. Körpergelenkstabilisierungsvorrichtung (1) gemäß einem der vorstehenden Ansprüche, **dadurch gekennzeichnet, dass** das Verbindungselement (50) Fasern, einen Riemen, einen Gurt, ein Seil, ein Kabel und/oder einen Draht aufweist.

12. Körpergelenkstabilisierungsvorrichtung (1) gemäß einem der vorstehenden Ansprüche, **dadurch gekennzeichnet, dass** das Verbindungselement (50) integral mit dem Wirkkörper (60) ausgebildet ist.

13. Körpergelenkstabilisierungsvorrichtung (1) gemäß einem der vorstehenden Ansprüche, **dadurch gekennzeichnet, dass** das Umlenkmittel (70) mindestens zwei Umlenkrollen zum Umlenken des Verbindungselements (50) umfasst, wobei die eine Umlenkrolle am Zugkörper (40) und die andere Umlenkrolle am Wirkkörper (60) oder an der Aufnahme (20) angeordnet ist.

14. Körpergelenkstabilisierungsvorrichtung (1) gemäß einem der vorstehenden Ansprüche, **dadurch gekennzeichnet, dass** die Interaktion zwischen dem Wirkkörper (60) und dem scherverdickenden Medium (30) derart konfiguriert ist, dass wenn eine Kraft mit einer Geschwindigkeit unterhalb eines Schwellwerts auf den Wirkkörper (60) wirkt, der Wirkkörper (60) durch das scherverdickende Medium (30) bewegbar ist, und dass wenn eine Kraft mit einer Geschwindigkeit größer gleich eines Schwellwerts auf den Wirkkörper (60) wirkt, das scherverdickende Medium (30) die Bewegung des Wirkkörpers (60) hemmt.

15. Körpergelenkstabilisierungsvorrichtung (1) gemäß einem der vorstehenden Ansprüche, **dadurch gekennzeichnet, dass** das scherverdickende Medium (30) Festkörper, insbesondere Polymere oder Pulver und/oder ein Fluid, insbesondere eine Paste oder ein Gel, oder eine Kombination daraus umfasst.

16. Körpergelenkstabilisierungsvorrichtung (1) gemäß einem der vorstehenden Ansprüche, **dadurch gekennzeichnet, dass** die Aufnahme (20) integral mit einem Rahmen zum Befestigen der Körpergelenkstabilisierungsvorrichtung (1) an einem Körperteil ausgebildet ist.

## Claims

1. A body joint stabilizing apparatus (1) for adaptively damping a body movement, comprising:
a receptacle (20), wherein the receptacle (20) is filled with a shear-thickening medium (30),
a tensioning body (40) that is movable relative to the receptacle (20),
wherein the tensioning body (40) is connectable to a body region of the user that is movable relative to another body region of the user, on which the receptacle (20) is fastenable,
an effector body (60) that is displaceably arranged in the receptacle (20) and provided for an interaction with the shear-thickening medium (30), and
a connection element (50) for transmitting forces between the receptacle (20) and the tensioning body (40),
**characterized in that**
the receptacle (20), the tensioning body (40) and the effector body (60) are connected to one another by way of the connection element (50), wherein the connection element (50) is guided via at least one deflection means (70) for the purpose of dividing the force acting on the connection element (50).

2. The body joint stabilizing apparatus (1) according to claim 1, **characterized in that** the connection element (50) comprises a first end (52) and a second end (54), and wherein the at least one deflection means (70) is guided in movable fashion on the connecting element between the first end (52) and the second end (54).

3. The body joint stabilizing apparatus (1) according to claim 1 or 2, **characterized in that** the first end (52) of the connection element (50) is connected to the effector body (60) or the tensioning body (40) and the second end (54) of the connection element (50) is connected to the receptacle (20) or the tensioning body (40).

4. The body joint stabilizing apparatus (1) according to one of the preceding claims, **characterized in that** the deflection means (70) deflects the connection element (50) at least once through an angle of between 150° and 190°.

5. The body joint stabilizing apparatus (1) as claimed in any one of the preceding claims, **characterized in that** the deflection means (70) deflects the connection element (50) at least once through substantially 180°.

6. The body joint stabilizing apparatus (1) according to one of the preceding claims, **characterized in that** the tensioning body (40) is arranged outside of the receptacle (20).

7. The body joint stabilizing apparatus (1) according to one of the preceding claims, **characterized in that** the deflection means (70) comprises a material with a low coefficient of friction.

8. The body joint stabilizing apparatus (1) according to one of the preceding claims, **characterized in that** the deflection means (70) comprises at least one deflection pulley.

9. The body joint stabilizing apparatus (1) according to one of the preceding claims, **characterized in that** the connection element (50) is tension-resistant.

10. The body joint stabilizing apparatus (1) according to one of the preceding claims, **characterized in that** the connection element (50) is embodied in the form of an elongate, flexible tension element.

11. The body joint stabilizing apparatus (1) according to one of the preceding claims, **characterized in that** the connection element (50) comprises fibers, a strap, a belt, a rope, a cable and/or a wire.

12. The body joint stabilizing apparatus (1) according to one of the preceding claims, **characterized in that** the connection element (50) is integral with the effector body (60).

13. The body joint stabilizing apparatus (1) according to one of the preceding claims, **characterized in that** the deflection means (70) comprises at least two deflection pulleys for deflecting the connection element (50), with one deflection pulley being arranged on the tensioning body (40) and the other deflection pulley being arranged on the effector body (60) or on the receptacle (20).

14. The body joint stabilizing apparatus (1) according to one of the preceding claims, **characterized in that** the interaction between the effector body (60) and the shear-thickening medium (30) are configured in such a way that the effector body (60) is movable through the shear-thickening medium (30) if a force acts on the effector body (60) with a speed below a threshold and that the shear-thickening medium (30) inhibits the movement of the effector body (60) if a force acts on the effector body (60) with a speed that is greater than or equal to a threshold.

15. The body joint stabilizing apparatus (1) according to one of the preceding claims, **characterized in that** the shear-thickening medium (30) comprises solids, in particular polymers or powders and/or a fluid, in particular a paste or a gel, or any combination thereof.

16. The body joint stabilizing apparatus (1) according to one of the preceding claims, **characterized in that** the receptacle (20) comprises an integral embodiment with a frame for fastening the body joint stabilizing apparatus (1) to a body part.

## Revendications

1. Dispositif de stabilisation de l'articulation du corps (1) pour amortir de manière adaptative un mouvement du corps, comprenant :
un logement (20), ledit logement (20) étant rempli d'un milieu dilatant (30),
un corps de traction (40) mobile par rapport au logement (20),
ledit corps de traction (40) peut être relié à une zone de corps de l'utilisateur qui est mobile par rapport à une autre zone de corps de l'utilisateur à laquelle le logement (20) peut être fixé sur,
un corps actif (60) disposé de manière coulissante dans le logement (20) pour interagir avec le milieu dilatant (30), et
un élément de liaison (50) pour la transmission des forces entre le logement (20) et le corps de traction (40),
**caractérisé en ce que**
le logement (20), le corps de traction (40) et le corps actif (60) sont reliés entre eux par l'intermédiaire de l'élément de liaison (50), ledit élément de liaison (50) étant guidé par au moins un moyen de renvoi (70) afin de répartir la force agissant sur l'élément de liaison (50).

2. Dispositif de stabilisation de l'articulation du corps (1) selon la revendication 1, **caractérisé en ce que** l'élément de liaison (50) présente une première extrémité (52) et une deuxième extrémité (54), ledit au moins un moyen de renvoi (70) étant guidé de manière mobile entre la première extrémité (52) et la deuxième extrémité (54) sur l'élément de liaison (50).

3. Dispositif de stabilisation de l'articulation du corps (1) selon la revendication 1 ou 2, **caractérisé en ce que** la première extrémité (52) de l'élément de liaison (50) est reliée au corps actif (60) ou au corps de traction (40) et la deuxième extrémité (54) de l'élément de liaison (50) au logement (20) ou au corps de traction (40).

4. Dispositif de stabilisation de l'articulation du corps (1) selon l'une des revendications précédentes, **caractérisé en ce que** le moyen de renvoi (70) dévie l'élément de liaison (50) au moins une fois selon un angle compris entre 150° et 190°.

5. Dispositif de stabilisation de l'articulation du corps (1) selon l'une des revendications précédentes, **caractérisé en ce que** le moyen de renvoi (70) dévie l'élément de liaison (50) au moins une fois d'un angle sensiblement égal à 180°.

6. Dispositif de stabilisation de l'articulation du corps (1) selon l'une des revendications précédentes, **caractérisé en ce que** le corps de traction (40) est disposé à l'extérieur du logement (20).

7. Dispositif de stabilisation de l'articulation du corps (1) selon l'une des revendications précédentes, **caractérisé en ce que** le moyen de renvoi (70) est constitué d'un matériau ayant un faible coefficient de frottement.

8. Dispositif de stabilisation de l'articulation du corps (1) selon l'une des revendications précédentes, **caractérisé en ce que** le moyen de renvoi (70) comprend au moins un rouleau de renvoi.

9. Dispositif de stabilisation de l'articulation du corps (1) selon l'une des revendications précédentes, **caractérisé en ce que** l'élément de liaison (50) est formé de manière rigide à la traction.

10. Dispositif de stabilisation de l'articulation du corps (1) selon l'une des revendications précédentes, **caractérisé en ce que** l'élément de liaison (50) est conçu sous la forme d'un élément de traction allongé et flexible.

11. Dispositif de stabilisation de l'articulation du corps (1) selon l'une des revendications précédentes, **caractérisé en ce que** l'élément de liaison (50) comprend des fibres, une sangle, une ceinture, une corde, un câble et/ou un fil.

12. Dispositif de stabilisation de l'articulation du corps (1) selon l'une des revendications précédentes, **caractérisé en ce que** l'élément de liaison (50) est formé intégralement d'un seul tenant avec le corps actif (60).

13. Dispositif de stabilisation de l'articulation du corps (1) selon l'une des revendications précédentes, **caractérisé en ce que** le moyen de renvoi (70) comprend au moins deux rouleaux de renvoi pour dévier l'élément de liaison (50), ledit un rouleau de renvoi étant disposé sur le corps de traction (40) et l'autre rouleau de renvoi sur le corps actif (60) ou sur le logement (20).

14. Dispositif de stabilisation de l'articulation du corps (1) selon l'une des revendications précédentes, **caractérisé en ce que** l'interaction entre le corps actif (60) et le milieu dilatant (30) est configurée de telle sorte que lorsqu'une force à une vitesse inférieure à une valeur seuil est appliquée sur le corps actif (60), le corps actif (60) est mobile par le milieu dilatant (30), et que lorsqu'une force à une vitesse égale à une valeur seuil agit sur le corps actif (60), le milieu dilatant (30) inhibe le mouvement du corps actif (60).

15. Dispositif de stabilisation de l'articulation du corps (1) selon l'une des revendications précédentes, **caractérisé en ce que** le milieu dilatant (30) comprend des solides, en particulier des polymères ou des poudres et/ou un fluide, en particulier une pâte ou un gel, ou une combinaison de ceux-ci.

16. Dispositif de stabilisation de l'articulation du corps (1) selon l'une des revendications précédentes, **caractérisé en ce que** le logement (20) est formé d'un seul tenant avec un cadre pour la fixation du dispositif de stabilisation de l'articulation du corps (1) à un élément du corps.
